(19) Europäisches Patentamt
European Patent Office
Office européen des brevets

(11) Numéro de publication: **0 109 909**
**B1**

# (12) FASCICULE DE BREVET EUROPEEN

(45) Date de publication du fascicule du brevet: **21.08.85**

(51) Int. Cl.[4]: **C 07 C 121/75**, C 07 C 69/65, A 01 N 53/00

(21) Numéro de dépôt: **83402236.0**

(22) Date de dépôt: **21.11.83**

(54) **Nouveaux dérivés de l'acide 2,2-diméthyl 3/2-fluoro 3-oxo 3(1,1-diméthyl éthoxy) 1-propényl cyclopropane carboxylique.**

(30) Priorité: **22.11.82 FR 8219516**

(43) Date de publication de la demande: **30.05.84 Bulletin 84/22**

(45) Mention de la délivrance du brevet: **21.08.85 Bulletin 85/34**

(84) Etats contractants désignés: **AT BE CH DE GB IT LI LU NL SE**

(56) Documents cités:
**EP - A - 0 041 021**
**EP - A - 0 050 534**
**FR - A - 2 185 612**

(73) Titulaire: **ROUSSEL-UCLAF, 35, boulevard des Invalides, F-75007 Paris (FR)**

(72) Inventeur: **Martel, Jacques, 15, rue Douvillez, F-93140 Bondy (FR)**
Inventeur: **Tessier, Jean, 30, rue Jean Moulin, F-94300 Vincennes (FR)**
Inventeur: **Teche, André, 15, rue Godot de Mauroy, F-75009 Paris (FR)**

(74) Mandataire: **Tonnellier, Marie-José et al, ROUSSEL-UCLAF 111, route de Noisy Boîte Postale no.9, F-93230 Romainville (FR)**

**EP 0 109 909 B1**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

BUNDESDRUCKEREI BERLIN

## Description

La présente invention concerne de nouveaux dérivés de l'acide 2,2-diméthyl 3/2-fluoro 3-oxo 3(1,1-diméthyl éthoxy) 1-propényl/ cyclopropane carboxylique, leur procédé de préparation et leur application dans la lutte contre les acariens parasites des végétaux.

L'invention a pour objet:

— le (1Rcis, E) 2,2-diméthyl 3/2-fluoro 3-oxo 3(1,1-diméthyl éthoxy) 1-propényl/ cyclopropane carboxylate de cyano (3-phénoxy 4-fluoro phényl) méthyle.

L'invention a plus spécialement pour objet:

— le (1Rcis, E) 2,2-diméthyl 3/2-fluoro 3-oxo 3(1,1-diméthyl éthoxy) 1-propényl/ cyclopropane carboxylate de (S) cyano (3-phénoxy 4-fluoro phényl) méthyle.

Les composés de l'invention font partie d'une famille de produits chimiques qui possèdent de très intéressantes propriétés pesticides décrites et revendiquées dans la demande de brevet européen 0 050 534. Ces composés sont connus pour pouvoir être utilisés dans la lutte contre les parasites des végétaux, les parasites des animaux à sang chaud et les parasites des locaux, pour lutter par exemple, contre les insectes, les acariens parasites des végétaux, pour lutter contre les nématodes ainsi que pour lutter contre les acariens parasites des animaux notamment, les tiques et les gales.

Or, il vient d'être trouvé que si les produits de la présente invention présentent les activités générales des produits de la demande précitée, ils sont doués d'une propriété acaricide vis-à-vis des acariens parasites des végétaux tout à fait exceptionnelle et totalement inattendue, par rapport à l'activité dans ce même domaine des produits du brevet précité.

C'est ainsi qu'il est démontré, dans la partie expérimentale figurant plus loin, que l'activité acaricide d'un produit de l'invention est double de celle de son analogue non fluoré, tandis qu'une telle différence n'existe pas entre deux dérivés analogues, l'un fluoré et l'autre non, appartenant tous deux au BE 0 050 534.

On se trouve donc en présence d'un phénomène de sélection que l'art antérieur ne laissait pas prévoir.

L'invention a donc tout spécialement pour objet les compositions destinées à lutter contre les acariens parasites des végétaux renfermant comme principe actif au moins l'un des produits de l'invention.

Les compositions de l'invention sont préparées selon les procédés usuels de l'industrie agrochimique.

Dans les compositions de l'invention, la ou les matières actives peuvent être additionnées éventuellement d'un ou plusieurs agents pesticides. Ces compositions peuvent se présenter sous forme de poudres, granulés, suspensions, émulsions, solutions, solutions pour aérosols, bandes combustibles, appâts ou autres préparations employées classiquement pour l'utilisation de ce genre de composés.

Outre le principe actif, ces compositions contiennent, en général, un véhicule et/ou un agent tensio-actif, non ionique, assurant, en outre, une dispersion uniforme des substances constitutives du mélange. Le véhicule utilisé peut être un liquide, tel que l'eau, l'alcool, les hydrocarbures ou autres solvants organiques, une huile minérale, animale ou végétale, une poudre telle que le talc, les argiles, les silicates, le kieselgühr ou un solide combustible tel que la poudre de tabu (ou marc de pyrèthre).

Les compositions de l'invention peuvent être additionnées éventuellement d'un ou plusieurs autres agents pesticides.

Pour obtenir de bons résultats acaricides, on utilise de préférence des poudres mouillables, pour pulvérisation foliaire contenant de 1 à 80 pour cent de principe actif, ou des liquides pour pulvérisation foliaire contenant de 1 à 500 g/l de principe actif. On peut également employer des poudres pour poudrage foliaire contenant de 0,05 à 3% de matière acitve.

Les composès de l'invention sont utilisés, de préférence à des dases comprises entre 1 et 100 g de matière active à l'hectare.

Pour exalter l'activité biologique des produits de l'invention, on peut les additionner à des synergistes classiques utilisés en pareil cas tel que le 1-(2,5,8-trioxadodécyl) 2-propyl 4,5-méthylènedioxy benzène (ou butoxyde de pipéronyle) ou la N-(2-éthyl heptyl) bicyclo/2,2-1/-5-heptène-2,3-dicarboximide, ou le pipéronyl-bis-2-(2'-n-butoxy éthoxy) éthylacétal (ou tropital).

L'invention a également pour objet un procédé de préparation caractérisé en ce que l'on soumet l'acide (1Rcis, E) 2,2-diméthyl 3/2-fluoro 3-oxo 3(1,1-diméthyl éthoxy) 1-propényl/ cyclopropane carboxylique ou un de ses dérivés fonctionnels à l'action de l'alcool $\alpha$-cyano 3-phénoxy 4-fluoro benzylique ou de l'alcool (S) $\alpha$-cyano 3-phénoxy 4-fluoro benzylique pour obtenir le composé recherché.

Le dérivé fonctionnel d'acide utilisé est de préférence un chlorure d'acide.

La réaction d'estérification peut être réalisée selon d'autres procédés. On peut par example, faire réagir l'acide ci-dessus avec l'alcool ci-dessus en présence de dicyclohexylcarbodiimide ou de diisopropylcarbodiimide.

L'invention a plus particulièrement pour objet un procédé de préparation des composés de l'invention caractérisé en ce que l'acide (1Rcis, E) 2,2-diméthyl 3/2-fluoro 3-oxo 3(1,1-diméthyl éthoxy) 1-propényl/ cyclopropane carboxylique, est préparé en faisant réagir, selon la réaction de Wittig, l'acide 2,2-diméthyl 3-formyl cyclopropane 1-carboxylique et le phosphonoacétate fluoré de formule:

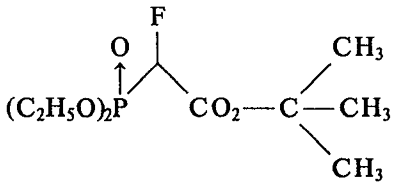

pour obtenir un mélange d'ácide recherché et de son isomère Z, puis en isolant du mélange l'isomère E.

Les isomères au niveau e la double liaison peuvent être séparés par des méthodes physiques telles que la chromatographie.

L'acide (1Rcis, E) 2,2-diméthyl 3/2-fluoro 3-oxo 3(1,1-diméthyl éthoxy) 1-propényl cyclopropane carboxylique obtenu lors de la mise en œuvre du procédé de l'invention est un produit nouveau et est en lui-même, un objet de la présente invention.

L'invention a tout spécialement pour objet ce produit à titre de produit chimique nouveau et notamment, à titre de produit intermédiaire nécessaire à la mise en œuvre du procédé de l'invention.

Les exemples suivants illustrent l'invention sans toutefois la limiter.

## Exemple 1

(1Rcis, E) 2,2-diméthyl 3/2-fluoro 3-oxo 3(1,1-diméthyl éthoxy) 1-propényl/ cyclopropane carboxylate de (S) cyano (3-phénoxy 4-fluoro phényl) méthyle

On verse en 10 minutes à une température comprise entre 5 et 10° C, une solution renfermant 2,4 g de dicyclohexylcarbodiimide, 80 mg de diméthylaminopyridine et 10 $cm^3$ de chlorure de méthylène dans une mélange renfermant 3 g d'acide (1Rcis, E) 2,2-diméthyl 3/2-fluoro 3-oxo 3(1,1-diméthyl éthoxy) 1-propényl cyclopropane carboxylique, 15 $cm^3$ de chlorure de méthylène et 3,3 g d'alcool (S) cyano (3-phénoxy 4-fluoro phényl) méthylique. On maintient le mélange réactionnel sous agitation pendant 15 minutes à +5° C puis, pendant 3 heures à la température ambiante. On filtre le précipité formé, rince et sèche le filtrat. Par évaporation à sec du filtrat, on obtient 6,4 g de produit recherché sous forme brute que l'on purifie par chromatographie sur silice en éluant par le mélange hexane-acétate d'éthyle 9-1 puis recristallise dans l'éther isopropylique pour obtenir 4,4 g de produit recherché fondant à 122° C.

$(\alpha)_D = +61° + 2,5°$ (c = 0,35% $CHCl_3$).

## Préparation 1

Acide (1Rcis, E) 2,2-diméthyl 3/2-fluoro 3-oxo 3(1,1-diméthyl éthoxy) 1-propényl/ cyclopropane carboxylique ou:

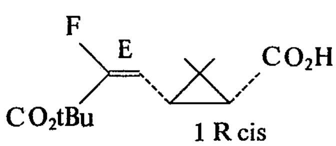

et son isomère Z correspondant.

On mélange 1,2 g de bromure de lithium, 600 mg d'hemi acétal interne de l'acide 2,2-diméthyl 3-formyl cyclopropane 1-carboxylique, 700 mg de phosphonoacétate fluoré de formule:

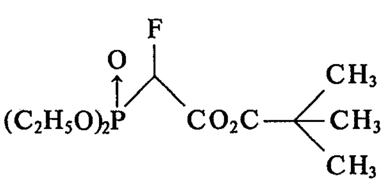

et 20 cm³ de tétrahydrofuran. On refroidit à −30° C puis introduit goutte à goutte une solution de 800 mg de tertbutylate de potassium et 5 cm³ de tétrahydrofuran. On laisse 45 minutes à −30° C, puis verse sur une solution d'acide isopropylique. On obtient une huile que l'on chromatographie sur silice en éluant par le mélange hexane-acétate d'éthyle-acide acétique (7-3-0,1). On obtient 550 mg d'acide pur, isomère E et 50 mg d'isomère Z.

Isomère E: spectre de RMN (ppm)

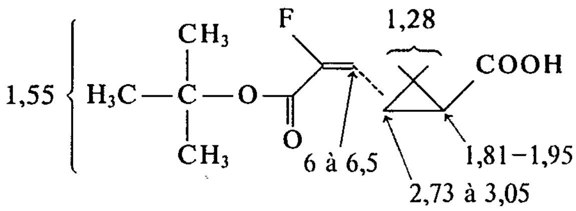


Isomère Z: spectre de RMN (ppm)
(F = 90° C) après recristallisation dans l'hexane.

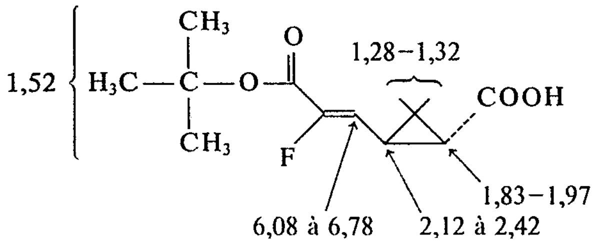


Exemple 2

Etude de l'activité acaricide

On a étudié l'activité acaricide du produit de l'exemple 1 ou produit A, de son analogue dérivé de l'alcool (3-phénoxy phényl) méthyle (ou produit B), du (1Rcis, E) 2,2-diméthyl 3/2-fluoro 2-éthoxy carbonyl/ éthényl/ cyclopropane-1-carboxylate de (3-phénoxy 4-fluoro phényl) méthyle (ou produit C) et de son analogue dérivé de l'alcool (3-phénoxy phényl) méthyllique (ou produit D).

Les produits B, C et D sont décrits dans les exemples 21, 11 et 1 de la demande de brevet européen 0 050 534.

Technique utilisée

On utilise des plants de haricots comportant 2 feuilles infestées de 25 femelles de Tétranychus Urticae par feuille et mis sous bonette aérée sous plafond lumineux en lumière constante. Les plants sont traités au pistolet Fisher: 4 ml de solution toxique par plant d'un mélange à volume égal d'eau et d'acétone. On laisse sécher pendant 12 heures puis on procède à l'infestation. Les contrôles de mortalité sont effectués 80 heures après.

Les résultats sont exprimés en concentration létale 50 ($CL_{50}$).
Le composé A a une CL 50 de 416 mg/hl.
Le composé B a une CL 50 de 807 mg/hl.
Le composé C a une CL 50 de 1870 mg/hl.
Le composé D a une CL 50 de 1816 mg/hl.

Conclusion

Le composé A a une activité double de celle de son analogue B, alors que le composé C a une activité comparable à celle de son homologue D.

L'activité du composé A est tout à fait surprenante.

## Exemple 3

## Exemples de compositions

### Exemple A

### Préparation d'un concentré soluble

On mélange d'une façon homogène:

- produit de l'exemple 1 0,25 g
- butoxyde de pipéronyle 1 g
- tween 80 0,25 g
- topanol A 0,1 g
- eau 98,4 g

### Exemple B

### Préparation d'un concentré émulsifiable

On mélange d'une façon homogène:

- produit de l'exemple 1 0,015 g
- butoxyde de pipéronyle 0,5 g
- topanol A 0,1 g
- tween 80 3,5 g
- xylène 95,885 g

### Exemple C

### Préparation d'un concentré émulsifiable

On effectue un mélange homogène de:

- produit de l'exemple 1 1,5 g
- tween 80 20 g
- topanol A 0,1 g
- xylène 78,4 g

**Revendications**

1. Le (1Rcis, E) 2,2-diméthyl 3/2-fluoro 3-oxo 3(1,1-diméthyl éthoxy) 1-propényl/ cyclopropane carboxylate de cyano 3-phénoxy 4-fluoro phényl méthyle.

2. Le diastéréoisomère suivant du composé défini à la revendication 1: le (1Rcis, E) 2,2-diméthyl 3/2-fluoro 3-oxo 3(1,1-diméthyl éthoxy) 1-propényl/ cyclopropane carboxylate de (S) cyano (3-phénoxy 4-fluoro phényl) méthyle.

3. Les compositions destinées à lutter contre les acariens parasites des végétaux renfermant comme principe actif au moins un produit défini à la revendication 1 ou 2.

4. Procédé de préparation des composés définis aux revendications 1 et 2 caractérisé en ce que l'on soumet l'acide (1Rcis, E) 2,2-diméthyl 3/2-fluoro 3-oxo 3(1,1-diméthyl éthoxy) 1-propényl/ cyclopropane carboxylique ou un de ses dérivés fonctionnels à l'action de l'alcool $\alpha$-cyano 3-phénoxy 4-fluoro benzylique ou de l'alcool (S) $\alpha$-cyano 3-phénoxy 4-fluoro benzylique pour obtenir le composé recherché.

5. Procédé de préparation selon la revendication 4 caractérisé en ce que l'acide (1Rcis, E) 2,2-diméthyl 3/2-fluoro 3-oxo 3(1,1)-diméthyl ethoxy) 1-propényl/ cyclopropane carboxylique est préparé en faisant réagir, selon la réaction de Wittig, l'acide 2,2-diméthyl 3-formyl cyclopropane 1-carboxylique et le phosphonoacétate fluoré de formule:

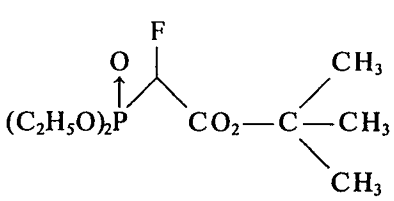

pour obtenir un mélange de l'acide recherché E et de son isomère Z, puis en isolant du mélange l'isomère E.

6. A titre de produit intermédiaire nécessaire à la préparation du composé de la revendication 1, l'acide (1Rcis, E) 2,2-diméthyl 3/2-fluoro 3-oxo 3(1,1-diméthyl éthoxy) 1-propényl cyclopropane carboxylique.

**Patentansprüche**

1. Cyano-3-phenoxy-4-fluor-phenylmethyl-(1R cis,E)-2,2-dimethyl-3-[2-fluor-3-oxo-3-(1,1-dimethyl-äthoxy)-1-propenyl]-cyclopropan-carboxylat.

2. Das der Verbindung gemäß Anspruch 1 entsprechende Diastereomere: (S)-Cyano-(3-phenoxy-4-fluor-phenyl)-methyl-(1Rcis,E)-2,2-dimethyl-3-[2-fluor-3-oxo-3-(1,1-dimethyl-äthoxy)-1-propenyl]-cyclopropan-carboxylat.

3. Zusammensetzungen für die Bekämpfung von parasitären Milben der Pflanzen, enthaltend als Wirkstoff zumindest ein Produkt gemäß Anspruch 1 oder 2.

4. Verfahren zur Herstellung der Verbindungen gemäß den Ansprüchen 1 und 2, dadurch gekennzeichnet, daß man die (1R cis,E)-2,2-Dimethyl-3-[2-fluor-3-oxo-3-(1,1-dimethyl-äthoxy)-1-propenyl]-cyclopropan-carbonsäure oder eines ihrer funktionellen Derivate der Einwirkung von $\alpha$-Cyano-3-phenoxy-4-fluor-benzyl-alkohol oder (S)-$\alpha$-Cyano-3-phenoxy-4-fluor-benzylalkohol aussetzt, um die gewünschte Verbindung zu erhalten.

5. Verfahren gemäß Anspruch 4, dadurch gekennzeichnet, daß man die (1Rcis,E)-2,2-Dimethyl-3-[2-fluor-3-oxo-3-(1,1-dimethyl-äthoxy)-1-propenyl]-cyclopropan-carbonsäure herstellt, indem man nach der Wittig-Reaktion 2,2-Dimethyl-3-formyl-cyclopropan-1-carbonsäure und das fluorierte Phosphonoacetat der Formel

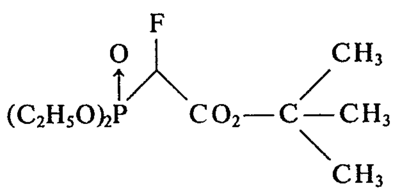

umsetzt, um ein Gemisch der gewünschten E-Säure und ihres Z-Isomeren zu erhalten und dann aus dem Gemisch das E-Isomere isoliert.

6. Als Zwischenprodukt für die Herstellung der Verbindung des Anspruchs 1 die (1R cis,E)-2,2-Dimethyl-3-[2-fluor-3-oxo-3-(1,1-dimethyl-äthoxy)-1-propenyl]-cyclopropan-carbonsäure.

**Claims**

1. (1R cis, E) 2,2-dimethyl-3[2-fluoro-3-oxo-3(1,1-dimethylethoxy)1-propenyl]cyclopropane carboxylate of cyano-3-phenoxy-4-fluorophenylmethyl.

2. The following diastereoisomer of the compound defined in Claim 1: (1R cis, E) 2,2-dimethyl-3[2-fluoro-3-oxo-3(1,1-dimethylethoxy)-1-propenyl]cyclopropane carboxylate of (S) cyano (3-phenoxy-4-fluorophenyl)methyl.

3. Compositions intended for combatting parasitic acarids of vegetation containing as active principle at least one product defined in claim 1 or 2.

4. Preparation process for compounds defined in the claims 1 and 2 characterized in that (1Rcis, E) 2,2-dimethyl 3[2-fluoro-3-oxo-3(1,1-dimethylethoxy)-1-propenyl]cyclopropane carboxylic acid or one of its functional derivatives is submitted to the action of $\alpha$-cyano-3-phenoxy-4-fluorobenzyl alcohol or (S) $\alpha$-cyano-3-phenoxy-4-fluorobenzyl alcohol so as to obtain the compound sought.

5. Preparation process according to Claim 4 characterized in that (1R cis, E) 2,2-dimethyl-3[2-fluoro-3-oxo-3(1,1-dimethylethoxy)-1-propenyl]cyclopropane carboxylic acid is prepared by making 2,2-dimethyl-3-formyl cyclopropane-1-carboxylic acid and fluorine phosphonoacetate with the formula:

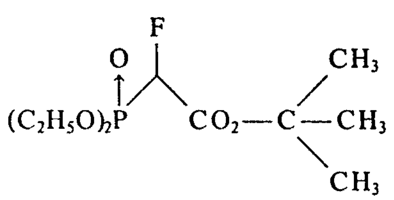

react according to Wittig's reaction so as to obtain a mixture of the sought acid E and its isomer Z, then isolating the isomer E from the mixture.

6. As an intermediate product necessary for the preparation of the compound of claim 1, (1R cis, E) 2,2-dimethyl-3[2-fluoro-3-oxo-3(1,1-dimethylethoxy)-1-propenyl cyclopropane carboxylic acid.